(19) Europäisches Patentamt
European Patent Office
Office européen des brevets

(11) EP 0 982 283 A2

(12) **EUROPÄISCHE PATENTANMELDUNG**

(43) Veröffentlichungstag:
**01.03.2000 Patentblatt 2000/09**

(21) Anmeldenummer: **99115960.9**

(22) Anmeldetag: **13.08.1999**

(51) Int. Cl.$^7$: **C07C 29/76**, C07C 31/18, C07C 37/20, C07C 39/10, B01D 61/42

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **24.08.1998 DE 19838425**

(71) Anmelder:
- **Degussa-Hüls Aktiengesellschaft**
**60311 Frankfurt am Main (DE)**
Benannte Vertragsstaaten:
**BE CH DE DK ES FI FR GB GR IE IT NL PT SE AT**
- **Servo Delden B.V.**
**7491 AE Delden (NL)**
Benannte Vertragsstaaten:
**BE CH AT**

(72) Erfinder:
- **Hörpel, Gerhard, Dr.**
**48301 Nottuln (DE)**
- **Kuppinger, Franz-Felix, Dr.**
**45768 Marl (DE)**
- **Schmidt, Friedrich Georg, Dr.**
**45721 Haltern (DE)**
- **van der Velden, Paulus Martinus, Dr.**
**7555 MK Hengelo (NL)**

# (54) Verfahren zur Abtrennung mehrfunktioneller Alkohole von wasserlöslichen Salzen aus wässrigen Systemen

(57) Die Erfindung betrifft ein Verfahren zur Abtrennung mehrfunktioneller Alkohole von wasserlöslichen Salzen aus wäßrigen Systemen unter Verwendung eines Elektrodialyseverfahrens.

EP 0 982 283 A2

## Beschreibung

Die vorliegende Erfindung betrifft ein Verfahren zur Abtrennung mehrfunktioneller Alkohole von wasserlöslichen Salzen aus wäßrigen Systemen.

Mehrfunktionelle Alkohole bzw. Polyole werden in großen Mengen für die unterschied-lichsten Einsatzgebiete hergestellt und finden z.B. als Wärmeübertragungsmittel, als Viskositätsveränderer, als Duftstoffkomponenten, als Tensidzwischenprodukte, als Salbengrundlagen, als Gefrierschutzmittel, als Additive für die Lackindustrie (Coatings), als Formtrennmittel, als Klebstoffe, als Weichmacher, als Ausgangsmaterial bei der Herstellung von Kunstharzen (z.B. Polyester oder Polyurethane) sowie als Schmier- bzw. Gleitmittel (Lubricants) Verwendung. Polymere mehrfunktionelle Alkohole, wie z.B. Polyvinylalkohol, werden z.B. als Schutzkolloide, als Suspensionsstabilisatoren, als Klebemittelbestandteile, als Pigmentbinder und als Verpackungsmaterialien verwendet (Ullmann's Encyclopedia of Industrial Chemistry, 5. Ed. (1985/1992), Vol. A1, Vol. A21).

Mehrfunktionelle Alkohole können auf unterschiedliche Art und Weise hergestellt werden, sei es durch Epoxidierungsreaktionen mit anschließender Hydrolyse (z.B. bei der Herstellung von Ethandiol), sei es durch Cannizzaro-Reaktionen (z.B. bei der Herstellung von Neopentylglykol) oder durch Verseifung von polymeren Vorläufern mit Laugen oder durch Umesterungen (z.B. bei der Herstellung von Polyvinylalkohol). In einigen Fällen fällt bereits bei der Herstellung, in anderen Fällen bei der anschließenden Aufbereitung und Anwendung ein aufzutrennendes Gemisch aus Salzen und dem mehrfunktionellen Alkohol an.

Es sind verschiedene Verfahren zur Extraktion der mehrfunktionellen Alkohole sowie Verfahren zum Auskristallisieren der Salze vorgeschlagen worden, um die wasserlöslichen mehrfunktionellen Alkohole von den ebenfalls wasserlöslichen Salzen abzutrennen.

Bei der Extraktion mit Lösungsmitteln für die mehrfunktionellen Alkohole, wie z.B. mit Amylalkohol, Cyclohexanol oder verschiedenen Estern, sind große Mengen an Extraktionsmittel erforderlich, die anschließend wieder z.B. durch Destillation vom gewünschten Produkt abgetrennt werden müssen.

Die US-PS 2468718 beschreibt ein Verfahren zum Trennen von Methylolalkanen von wasserlöslichen Salzen durch Extraktion der Methylolalkane mit einem niedrigsiedenden, wasserlöslichen Keton.

In Ullmann, Band 7, 4. Auflage, S. 231 (1974) wird beschrieben, daß das Gemisch aus Polyolen und Salzen durch Extraktion mit Lösungsmitteln, wie Amylalkohol, Cyclohexanol oder Essigester, und anschließender Destillation der Polyole aufgearbeitet werden kann. Diese Extraktionsverfahren erfordern jedoch die Verwendung großer Mengen an Extraktionsmitteln, die nachfolgend entfernt werden müssen. Dabei werden intensiv gefärbte Produkte erhalten, die eine Vielzahl von Nebenprodukten enthalten und die hauptsächlich wegen ihrer nachteiligen Farbe einer weiteren Destillation unterworfen werden müssen.

Die Abtrennung der Nebenprodukte ist deshalb von Bedeutung, weil diese in den nachfolgenden Anwendungen zu nicht beherrschbaren Störungen führen würden. So beeinflussen gefärbte Nebenprodukte in der Lackanwendung die Reproduzierbarkeit der Coatings nachteilig. Weiterhin wirken sich Reste von Salzen störend auf eventuelle weitere Umsetzungen der mehrfunktionellen Alkohole, z.B. bei der Herstellung der ent-sprechenden Ester, aus.

Es ist ebenfalls möglich, das Gemisch aus Polyolen und Salzen aufzukonzentrieren und die Salze auszukristallisieren (CN-A-1076185). Aber auch bei diesem Verfahren werden gefärbte Produkte erhalten. Hierzu kommt, daß im Kristallisat beträchtliche Mengen an Polyolen verbleiben, die sich nur unter deutlichen Ausbeuteverlusten auswaschen lassen.

Aufgabe der vorliegenden Erfindung ist es, ein verbessertes Verfahren zur Abtrennung mehrfunktioneller Alkohole von wasserlöslichen Salzen aus wäßrigen Systemen bereitzustellen, das kostengünstig, d.h. mit geringem Energieverbrauch, durchgeführt werden kann, das ohne Einsatz von Lösungsmitteln auskommt und somit umwelt-freundlich durchgeführt werden kann, und mit dem farblose Polyole erhalten werden,
die ohne weitere Reinigungsprozesse direkt weiterverarbeitet werden können.

Diese Aufgabe wird erfindungsgemäß durch ein Verfahren zur Abtrennung mehrfunktioneller Alkohole von wasserlöslichen Salzen aus wäßrigen Systemen gelöst, das dadurch gekennzeichnet ist, daß als Trennverfahren ein Elektrodialyseverfahren verwendet wird, mit der Maßgabe, daß die mehrfunktionellen Alkohole nicht Trimethylolpropan umfassen.

Die mehrfunktionellen wasserlöslichen Alkohole, die erfindungsgemäß eingesetzt werden können, umfassen:

a) Diole der allgemeinen Formel HO-$R_1$-OH, wobei $R_1$ bedeutet:

1. eine lineare aliphatische Einheit, wie z.B. -$C_2H_4$-, -$C_3H_6$-, -$C_4H_8$- oder -$C_6H_{12}$- (z.B. Ethylenglycol, 1,2- oder 1,3-Propylenglycol und 1,2-, 1,3-oder 1,4-Butandiol),

2. eine lineare ungesättigte aliphatische Einheit, wie z.B. -$C_4H_6$-, -$C_4H_4$-und -$C_6H_{10}$- (z.B. 2,3-Buten-1,4-diol, 2,3-Butin-1,4-diol und 2,3- oder 3,4-Hexen-1,6-diol),

3. eine verzweigte aliphatische Einheit, wie z.B. $-C_4H_8-$, $-C_5H_{10}-$ und $-C_6H_{12}-$(z.B. 2-Methyl-propan-1,2-diol, 2-Methyl-propan-1,3-diol, 2,2-Dimethyl-propan-1,3-diol, 2-Methyl-butan-1,2-diol, 2-Methyl-butan-1,3-diol, 2-Methyl-butan-1,4-diol, 2,3-Dimethyl-butan-1,2-diol, 2,3-Dimethyl-butan-1,4-diol, 2,2-Dimethyl-butan-1,3-diol und 2,2-Dimethyl-butan-1,4-diol),

4. eine verzweigte ungesättigte aliphatische Einheit, wie z.B. $-C_5H_8-$ und $-C_6H_{10}-$ (z.B. 2-Methyl-2,3-buten-1,4-diol und 2,2-Dimethyl-3,4-penten-1,5-diol),

5. eine cyclische oder alicyclische aliphatische Einheit, wie z.B. $-C_5H_{10}-$, $-C_6H_{12}-$ und $-C_7H_{14}-$ (z.B. 1,2-Cyclopentandiol, 1,3-Cyclopentandiol, 1,2-Cyclohexandiol, 1,3-Cyclohexandiol und 1-Methyl-1,2-cyclopentandiol),

6. eine cyclische oder alicyclische ungesättigte aliphatische Einheit, wie z.B. $-C_5H_8-$, $-C_6H_{10}-$ und $-C_7H_{12}$ (z.B. 1,2-Cyclopenten-3,4-diol, 1,2-Cyclopenten-3,5-diol, 1,2-Cyclohexen-3,4-diol, 1,2-Cyclohexen-3,5-diol, 1,2-Cyclohexen-4,5-diol, 1,2-Cyclohexen-3,6-diol, 1,2,3,4-Cyclohexadien-5,6-diol und 1-Methyl-1,2-cyclopentendiol),

7. eine aromatische Einheit, wie z.B. $-C_6H_4-$ und $-C_6H_3(CH_3)-$ (z.B. 1,2-Dihydroxybenzol, 1,3-Dihydroxybenzol, 1,2-Dihydroxy-3-methylbenzol und 1,3-Dihydroxy-2-methylbenzol), wobei auch weitere Substituenten am aromatischen Ringsystem oder an alicyclischen Seitenketten vorhanden sein können,

8. eine aromatische heterocyclische Einheit, wie z.B. $-C_5H_3N-$ (z.B. 2,4-Dihydroxy-pyridin), und

9. eine Einheit, umfassend eine Kombination der zuvor genannten Einheiten (a) 1 bis 8.

b) Monoglyceride der allgemeinen Formel $CH_2OR_r-CHOR_s-CH_2OR_t$, worin zwei der Gruppen $R_r$, $R_s$ und $R_t$ jeweils Wasserstoff bedeuten und die andere Gruppe ist ein gegebenenfalls ungesättigter Monocarbonsäurerest mit 1 bis 22 Kohlenstoffatomen, wie z.B. $CH_2OH-CHOH-CH_2O-CO-C_{17}H_{35}$,

c) oligomere und polymere Etherdiole der allgemeinen Formel $HO-[(-CR_2R_3)_n-O]_m-H$, mit $n \geq 2$ und $m \geq 2$, worin $R_2$ und $R_3$ jeweils unabhängig voneinander Wasserstoff oder eine aliphatische Gruppe, wie z.B. - $CH_3$ oder $-C_2H_5$, bedeuten. Beispiele dafür umfassen $HO-CH_2-CH_2-O-CH_2-CH_2-OH$ (Diethylenglycol), $HO[CH_2CH_2O]_{30}H$ (Polyethylenglycol mit einem mittleren Molekulargewicht von 1338) oder $HO[CH_2CH(CH_3)O]_{30}H$ (Polypropylenglycol mit einem mittleren Molekulargewicht von 1758),

d) Triole und höherfunktionelle Alkohole der Formel $R_4(OH)_x$ mit $x \geq 3$, worin $R_4$ bedeutet:

1. eine lineare aliphatische Einheit, wie z.B $-C_3H_5-$, $-C_4H_7-$, $-C_5H_9-$ oder $-C_5H_8-$ (z.B. Glycerin, 1,2,3-Butantriol, 1,2,4-Butantriol und 1,2,3-Pentantriol),

2. eine lineare ungesättigte aliphatische Einheit, wie z.B. $-C_5H_7-$, $-C_6H_9-$oder $-C_6H_8-$ (z.B. 1-Penten-3,4,5-triol, 1-Hexen-3,4,5-triol, 2-Hexen-1,4,5-triol und 3-Hexen-1,2,5,6-tetrol),

3. eine verzweigte aliphatische Einheit, wie z.B. $-C_4H_7-$ oder $-C_5H_8-$(z.B. 2-Methyl-1,2,3-propantriol und Pentaerythrit),

4. eine verzweigte ungesättigte aliphatische Einheit, wie z.B. $-C_5H_7-$ und $-C_6H_9-$ (z.B. 2-Methylol-2-buten-1,4-diol und 2-Methylol-2-penten-1,5-diol),

5. eine cyclische oder alicyclische aliphatische Einheit, wie z.B. $-C_5H_7-$, $-C_6H_9-$, $-C_6H_8-$ und $-C_7H_{11}-$ (z.B. 1,2,3-Cyclopentantriol, 1,2,3-Cyclohexantriol, 1,2,3,4-Cyclohexantetrol, 6-Methyl-1,2,3-cyclohexantriol und 6-Methylol-1,3-cyclohexandiol);

6. eine cyclische oder alicyclische ungesättigte aliphatische Einheit, wie z.B. $-C_5H_5-$ und $-C_6H_7-$ (z.B. Cyclopenten-3,4,5-triol und Cyclohexen-3,4,5-triol),

7. eine aromatische Einheit, wie z.B. $-C_6H_3-$ und $-C_6H_2(CH_3)-$ (z.B. 1,2,3-Trihydroxybenzol und 2,3,4-Trihydroxytoluol),

8. eine aromatische heterocyclische Einheit, wie z.B. $-C_5H_2N-$ (z.B. 2,3,4-Trihydroxypyridin), und

9. eine Einheit, umfassend eine Kombination der zuvor genannten Einheiten (d) 1 bis 8, sowie

e) polymere mehrfunktionelle Alkohole, wie z.B. Polyvinylalkohol, Polysaccharide und verzweigte oder dendrimerartige Polyetherpolyole mit z.B. der folgenden Struktur

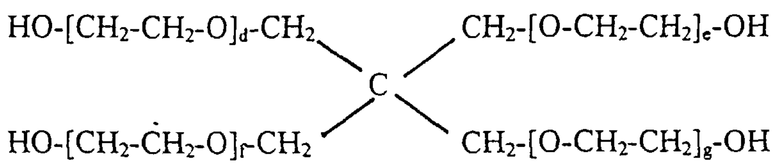

worin d, e, f und g unabhängig voneinander eine ganze Zahl ≥ 1 bedeuten.

Die Verwendung der Elektrodialyse zur Meerwasserentsalzung, zur Solegewinnung, zur Trinkwassergewinnung, zur Regulierung des Härtegrades von Wasser, zur Molkeent-salzung in der Lebensmittelindustrie, zur Verhinderung der Weinsteinabscheidung bei der Weinherstellung oder zur Rückgewinnung werrvoller Stoffe aus galvanischen Abwässern ist bekannt (Römpp Chemie Lexikon, Band 2. 10. Auflage, S. 1113-1114 (1997)). Bei diesen Anwendungen handelt es sich jedoch immer um wäßrige Systeme mit einem relativ geringen Salzgehalt (< 5 Gew.%).

Die Elektrodialyse ist ein Trennverfahren, bei dem die Wanderung von Ionen durch permselektive Membranen mit einer angelegten elektrischen Gleichspannung beschleunigt wird. Bei der Elektrodialyse werden Ionen unter der Einwirkung eines elektrischen Feldes durch Membranen transportiert. Wenn in der Dialysevorrichtung die Ionenaustauschermembranen so angeordnet werden, daß sich abwechselnd je eine Anionenaustauschermembran und eine Kationenaustauschermembran zwischen zwei Elektroden befinden und die Zelle in schmale Kammern teilen, so werden bei entsprechender Schaltung salzärmere und salzreichere Volumenströme erhalten, denn bei Stromdurchgang können die Kationen nur die Kationenaustauschermembranen passieren und die Anionen nur die Anionenaustauschermembranen. Die Anreicherung erfolgt gegen den Konzentrationsgradienten. Das heißt, durch Hintereinanderschalten mehrerer anionen- und kationenselektiver Ionenaustauschermembranen läßt sich die Entionisierung der zu dialysierenden Flüssigkeit bei gleichzeitiger Anreicherung der Ionen in den Konzentratkammern erreichen.

Im Gegensatz zu den anderen bekannten Membranverfahren wie Membranfiltration, Umkehrosmose oder Gaspermeation, bei denen die Fluide aufgrund von Druck- oder Konzentrationsunterschieden getrennt werden, kann bei der Elektrodialyse mit dem elektrischen Strom eine gezielt wirkende Trennkraft aufgebracht werden. Dadurch können geladene Bestandteile selektiv in der Lösung bewegt werden. Die zur Trennung verwendeten Ionenaustauschermembranen sind organische Polymermembranen mit funktionellen, ladungstragenden Gruppen und wirken ähnlich einem elektrischen Gleichrichter. Ionenaustauschermembranen erlauben nur den Durchtritt einer Ionenart, während die entgegengesetzt geladenen Ionen am Durchtritt gehindert werden. Der Aufbau eines Elektrodialysemoduls entspricht in etwa dem einer Kammerfilterpresse, d.h. es werden über interne Zuleitungskanäle in den Modulrahmen zwei oder drei Lösungen separat zugeführt, die dann, durch die Ionenaustauschermembranen voneinander getrennt, die jeweiligen Rahmenhohlraume durchströmen. Mittels der angelegten elektrischen Spannung werden Ionen aus den einzelnen Kammern durch die Ionenaustauschermembranen transportiert. Durch geeignetes Hintereinanderschalten von Anionen- (A) und Kationenaustauschermembranen (K), d.h. Membranen, die entweder nur Anionen oder nur Kationen passieren lassen, können Lösungen entsalzt, aufkonzentriert oder unerwünschte Ionen gegen andere ausgetauscht werden.

Ein Beispiel für eine Apparatur, die erfindungsgemäß zur Abtrennung mehrfunktioneller Alkohole von wasserlöslichen Salzen verwendet werden kann ist in Figur 1 gezeigt. Die Apparatur besteht aus einem Elektrodialysemodul mit drei separaten Kreisläufen (einem Salzabgeberkreislauf, einem Salzaufnehmerkreislauf sowie einem Elektrodenspülkreislauf) und den dazugehörigen Vorlagegefäßen Um eine große Membranfläche zu realisieren, wurden fünf aufeinanderfolgende Zelleinheiten eingebaut.

In Fig. 1 bedeuten:

M bzw. $M^+$ = Metallionen, X bzw. $X^-$ = Anionen, Polyol = Polyfunktioneller Alkohol,
K = Kationenaustauschermembran und A = Anionenaustauschermembran

Zum Austrag der an den Elektroden entstehenden Reaktionsgase werden die Endkammern von einer Elektrodenspüllösung durchströmt. Diese Elektrodenspüllösung sollte sich mit den Elektroden inert verhalten, einen geringen elektrischen Widerstand besitzen und keine Fremdionen in die Salzaufnehmer- und Salzabgeberkreisläufe abgeben.

Es wurde nun überraschenderweise gefunden, daß die Elektrodialyse ebenfalls dazu verwendet werden kann, um ein Gemisch aus wasserlöslichen Polyolen und darin gelösten wasserlöslichen Salzen aufzutrennen, selbst wenn die Salze in hoher Konzentration (> 50 Gew.-%) vorliegen. Dabei lassen sich hohe Selektivitäten (S > 250) erreichen.

Die Selektivität der Auftrennung zwischen der ursprünglich salzhaltigen und danach entsalzten Lösung sowie der konzentrierten Lösung bezüglich der neutralen Komponente mehrfunktioneller Alkohol wird wie bei sonstigen Membranverfahren definiert:

$$S =([\text{Polyol}]_{\text{Konz}}[\text{Salz}]_{\text{Konz}})/([\text{Polyol}]_{\text{Dil}}[\text{Salz}]_{\text{Dil}})$$

Dabei bedeuten:

| | |
|---|---|
| Dil: | Salzabgeberlösung |
| Konz: | Salzaufnehmerlösung |
| [Polyol]: | Gewichtsanteil Polyol der Lösung |
| [Salz]: | Gewichtsanteil der Salze in der Lösung |

Die Selektivität des Salztransports wird durch die Stromausbeute beschrieben, d.h. die tatsächlich transportierte Stoffmenge an Salz ($N_{gemessen}$ in Mol) bezogen auf die durch den elektrischen Ladungstransport (elektrischer Strom) maximale mögliche Menge ($N_{theoretisch}$).

$$\eta = N_{gemessen}/N_{theoretisch}$$

Bei der Entsalzung der alkoholischen Lösung kann trotz des hohen Salzgehaltes eine Stromausbeute von über 95% erreicht werden.

Die in dem erfindungsgemäßen Verfahren verwendete Elektrodialysevorrichtung besteht aus handelsüblich erhältlichen Elektrodialysemodulen mit ebenfalls handelsüblich erhält-lichen Anionen- und Kationenaustauschermembranen. Hierbei muß gegebenenfalls die Anionenaustauschermembran nach den Kriterien a) geringer Widerstand, b) hohe Selektivität in Bezug auf das Anion und c) geringer Lösungsmittelfluß ausgesucht werden.

Die Elektrodialysevorrichtung kann einen Salzabgeberkreislauf und einen Salzaufnehmerkreislauf umfassen, wie in Figur 1 gezeigt.

Die Mischung aus mehrfunktionellen Alkoholen und Salzen, wie sie z.B als Rohprodukt aus der basenkatalysierten Aldoladdition eines längerkettigen aliphatischen Aldehyds mit einem kürzerkettigen aliphatischen Aldehyd und anschließender Cannizzaro-Reak-tion in Gegenwart stöchiometrischer Mengen an Base (z.B. Alkalihydroxid) entsteht, kann unmittelbar dem Salzabgeberkreislauf einer Elektrodialysevorrichtung gemäß Figur 1 zugeführt werden. Es ist dabei unerheblich, wie hoch die Konzentration der Einzel-komponenten in der Mischung ist, solange die Mischung pumpbar ist.

Der pH-Wert der Salzabgeberlösung wird mit einer Säure oder einer Base (bevorzugt mit der gleichen Base, die bei der Herstellung der Polyole verwendet wurde) annähernd neutral eingestellt. Saure bzw. alkalische pH-Werte sind möglich, solange die Stabilität der Membranen hiervon nicht berührt wird. Bevorzugt sind pH-Werte im Bereich von 4 bis 10.

Die Obergrenze der Temperatur der Salzabgeberlösung wird durch die Stabilität der Ionenaustauschermembranen bestimmt; die Untergrenze der Temperatur wird durch die Viskosität bzw. die Pumpbarkeit des Mediums bestimmt. Die Temperatur wird jedoch bevorzugt auf einen Wert im Bereich von 10 bis 50°C eingestellt. Dabei muß berück-sichtigt werden, daß sich die Salzabgeberlösung während des Trennprozesses erwärmt.

Die Salzaufnehmerlösung in dem Salzaufnehmerkreislauf der Elektrodialysevorrichtung besteht bevorzugt aus Wasser oder einer wäßrigen Salzlösung. Der pH-Wert der Salz-aufnehmerlösung wird vorzugsweise auf einen Wert im Bereich von 4 bis 10 eingestellt, und die Temperatur der Lösung liegt bevorzugt im Bereich von 10 bis 50°C. Auch in diesem Fall wird die Obergrenze der Konzentration des Mediums durch die Pumpbarkeit der Lösung bestimmt. Wichtig ist auch, daß das Löslichkeitsprodukt der durch die Mem-branen permeierenden Anionen und Kationen in der Salzaufnehmerlösung nicht über-schritten wird. Salzablagerungen im Salzaufnehmerkreislauf können zu irreversiblen Schäden an der gesamten Vorrichtung, insbesondere an den Membranen führen.

Die Stromdichte bei der Elektrodialyse liegt bevorzugt im Bereich von 50 bis 750 A/m$^2$, besonders bevorzugt im Bereich von 150 bis 250 A/m$^2$. Die Stromausbeute kann unter optimalen Prozeßbedingungen über 95% betragen. Die Grenzstromdichte muß an die Salzkonzentration in dem zu entsalzenden Gemisch angepaßt werden und kann durch den Fachmann leicht ermittelt werden. Die Grenzstromdichte als Funktion der Konzen-tration bestimmt somit die

Regelung der Stromdichte während des Trennprozesses.

In der erfindungsgemäß verwendeten Elektrodialysevorrichtung werden bevorzugt Elektrodenspüllösungen eingesetzt, wie in Figur 1 gezeigt. Die Elektrodenspüllösungen stellen sicher, daß keine Reaktion der Elektroden mit den Stoffen der Lösung stattfindet, daß die bei der Elektrodenreaktion entstehenden Gase ausgetragen werden können, und daß durch eine hohe Leitfähigkeit der elektrische Widerstand verringert und damit der Energiebedarf der Elektrodenreaktion minimiert wird. Die Elektrodenspüllösung sollte, wenn möglich, die gleichen Kationen wie die anderen Salzlösungen enthalten, um ein Eindringen weiterer Kationen in den Prozeß zu vermeiden. Als Elektrodenspül-lösungen können wäßrige Lösungen anorganischer Salze eingesetzt werden.

Der Verlauf des Trennprozesses kann über die Leitfähigkeit der Salzabgeber- und Salzaufnehmerlösung verfolgt werden. Die Leitfähigkeit muß zu den analytisch bestimmten Gehalten an Polyol bzw. Salz korreliert werden, d.h., daß bei einer gegebenen Leitfähigkeit mit Hilfe anderer analytischer Methoden (z.B. Hochleistungsflüssigchromatographie, HPLC, Gaschromatographie, GC) die tatsächliche Konzentration an Polyol, Nebenprodukten und Salz bestimmt werden muß. Im allgemeinen reicht es aus, die Elektrodialyse so lange durchzuführen, bis die Leitfähigkeit der Salzabgeberlösung auf etwa 2 μS/cm abgefallen ist. Die Reinigung des gesamten Elektrodialysemoduls hängt von der Trennaufgabe ab. Für die Reinigung des Systems ist es ausreichend, das Modul alle zwei Wochen etwa 2 Stunden lang mit warmem VE-Wasser zu spülen.

Bei der basisch katalysierten Reaktion (z.B. mit Kalilauge oder Natronlauge) von längerkettigen aliphatischen Aldehyden (z.B. Butyraldehyd) mit kürzerkettigen aliphatischen Aldehyden (z.B. Formaldehyd) entstehen die Polyole nicht als Reinsubstanzen, sondern je nach Prozeßführung als Gemisch des jeweiligen Zielproduktes neben dem Dimeren des Zielproduktes und weiteren OH-funktionellen Verbindungen sowie dem entsprechenden Salz der Säure aus der Cannizzaro-Reaktion. Wird dieses Rohprodukt mittels Elektrodialyse entsalzt, bleibt eine wäßrige, farblose Lösung des Polyolgemisches zurück, die als solche oder auch in aufkonzentrierter Form ohne weitere Reinigungsprozesse weiteren Reaktionen unterworfen werden kann, z.B. Kondensationsreaktionen, wie Veresterungen mit z.B. Ölsäure zur Herstellung von Lubricants, oder mit Acrylsäure zur Herstellung von Coatingadditiven.

Sollten dennoch die mehrfunktionellen Alkohole aus dem Gemisch als Reinsubstanzen isoliert werden, stehen die destillativen Stofftrennungsmethoden zur Verfügung.

Die folgenden Beispiele erläutern die Erfindung.

<u>Beispiele</u>

Es wurden verschiedene hochkonzentrierte, wäßrige Lösungen polyfunktioneller Alkohole mit entsprechend hohem Salzanteil aufgearbeitet. Im Salzabgeberkreislauf wurde ein Gemisch aus Alkohol, Salz und Wasser vorgelegt. Die Aufnehmerphase enthielt zu Beginn eine geringe Menge des abzutrennenden Salzes, um eine gewisse Mindestleitfähigkeit zu erzeugen. Die Elektrodenspüllösungen wurden nach den zuvor beschriebenen Kriterien für den jeweiligen Anwendungsfall ausgewählt. In der Regel waren es mittelkonzentrierte Lösungen mit den gleichen Salzen, die auch aus der wäßrigen Alkohollösung entfernt werden sollten.

Die eingesetzten Alkohole waren

- Diole: (Ethandiol (Ethylenglykol), 1,4-Butandiol, Diethylenglykol),
- Triole: (Glycerin, Neopentylglykol, Pentaerythritol) sowie zwei
- Polyalkohole (Polyvinylalkohol, Polyethylenalkohol) mit einem mittleren Molekulargewicht von ca. 1500 g/Mol.

Außer diesen aliphatischen Alkoholen wurde noch zusätzlich 1,3-Dihydroxybenzol (Resorcinol) als Beispiel für einen mehrwertigen aromatischen Alkohol untersucht.

Soweit es die Löslichkeit der Alkohole im Wasser zuließ, wurde jeweils eine 40%-ige Lösung eingewogen. Lediglich bei Pentaerythritol konnte nur eine 6%-ige Lösung angesetzt werden. Der Salzanteil variierte von 5 bis 20%, der Rest war dann Wasser. Bei den Salzen handelte es sich sowohl um

- anorganische Salze wie NaCl und $Na_2SO_4$ als auch um
- Natrium- und Kaliumsalze kurzer und längerkettiger organischer Säuren (Kaliumacetat, Natriumformiat sowie Natriumethylhexansäure)

Tabelle 1

| Alkohole | | Salze | |
|---|---|---|---|
| Bezeichnung | Kurzbezeichnung | Bezeichnung | Kurzbezeichnung |
| Ethandiol | A1 | Natriumchlorid | S1 |
| 1,4-Butandiol | A2 | Kaliumacetat | S2 |
| Diethylenglykol | A3 | Natriumsulfat | S3 |
| Glycerin | A4 | Natriumformiat | S4 |
| Neopentylglykol | A5 | Natrium-Ethylhexansäure | S5 |
| Pentaerythritol | A6 | | |
| 1,3-Dihydroxybenzol | A7 | | |
| Polyethylenglykol | A8 | | |
| Polyvinylalkohol | A9 | | |

Die in Figur 1 dargestellte Apparatur bestand aus einem Elektrodialysemodul der Fa. Stantech mit drei separaten Kreisläufen und den dazugehörenden Vorlagegefäßen. In das Modul, das eine effektive Membranfläche von 100 $cm^2$ aufwies, wurden fünf aufeinanderfolgende Zelleinheiten eingebaut. Die verwendeten Membranen mit den Bezeichnungen C66-10F und AHA-2 stammten von der Fa. Tokuyama Soda.

Die genauen Gewichtsanteile mit den entsprechenden Kombinationen aus Alkohol, Salz und Wasser sind in Tabelle 2 aufgeführt.

**Tabelle 2**

| Alkohol | M(Alkohol) | w(Alkohol) | Salz | M(Salz) | w(Salz) | Wasser |
|---|---|---|---|---|---|---|
| | g/Mol | % | | g/Mol | % | % |
| A1 | 62 | 40,0 | S1 | 58,5 | 12 | 48,00 |
| A2 | 90 | 40,0 | S2 | 98,0 | 20 | 40,00 |
| A3 | 106 | 38,6 | S3 | 146,0 | 5 | 56,37 |
| A8 | 1500 | 40,0 | S1 | 58,5 | 12 | 48,00 |
| A5 | 104 | 40,0 | S4 | 68,0 | 14 | 46,00 |
| A4 | 92 | 40,0 | S5 | 167,0 | 5 | 55,00 |
| A6 | 136 | 6,0 | S4 | 68,0 | 14 | 80,00 |
| A7 | 110 | 36,6 | S3 | 146,0 | 16,5 | 46,90 |
| A9 | 20000 | 5,0 | S1 | 58,5 | 5 | 90,00 |

Ausgangskonzentrationen in Gewichtsprozent und Molmassen der untersuchten Alkohol-Salz-Lösungen.

Die Konzentrationen der Elektrodenspüllösungen waren für die $Na_2SO_4$-Lösung 10%-ig und für die $K_2CO_3$- und $Na_2CO_3$-Lösungen 20%-ig. Sie sind in Tabelle 3 mit den dazu-gehörigen Alkoholen und Salzen der Entsalzungslösung angegeben. Anstelle von Chloriden, die bei der Elektrodenreaktion zu elementarem Chlor reduziert würden, wur-den Carbonate in den Spüllösungen verwendet.

**Tabelle 3**

| Abgeberkreislauf | | Anode | | Kathode | |
|---|---|---|---|---|---|
| Alkohol | Salz | Salz | % | Salz | % |
| A1 | S1 | $Na_2CO_3$ | 20 | $Na_2CO_3$ | 20 |
| A2 | S2 | $K_2CO_3$ | 20 | $K_2CO_3$ | 20 |
| A3 | S3 | $Na_2SO_4$ | 10 | $Na_2SO_4$ | 10 |
| A8 | S1 | $Na_2CO_3$ | 20 | $Na_2CO_3$ | 20 |
| A5 | S4 | $Na_2CO_3$ | 20 | $Na_2CO_3$ | 20 |
| A4 | S5 | $Na_2CO_3$ | 20 | $Na_2CO_3$ | 20 |
| A6 | S4 | $Na_2CO_3$ | 20 | $Na_2CO_3$ | 20 |
| A7 | S3 | $Na_2SO_4$ | 10 | $Na_2SO_4$ | 10 |
| A9 | S1 | $Na_2CO_3$ | 20 | $Na_2CO_3$ | 20 |

Zusammensetzung und Konzentration der Elektrodenspüllösungen mit den Alkoholen und Salzen des Abgeberkreislaufes.

Exemplarisch sind in den Figuren 2 bis 5 der Strom/Spannungsverlauf sowie die Leitfähigkeitsänderungen in der Salzabgeber- und Salzaufnehmerlösung für die Entsalzung von S1 aus A8 dargestellt.

Die konzentrierte Salz/Alkohollösung befindet sich im Abgeberkreislauf, die verdünnte Salzlösung im Aufnehmerkreislauf. Sobald eine Spannung an die Elektroden angelegt wird, fließt durch Ionenleitung ein elektrischer Strom.

Bei konstanter Spannung nimmt die Stromstärke infolge der Leitfähigkeitsabnahme im Abgeberkreislauf stetig ab. Die Zunahme der Leitfähigkeit im Aufnehmerkreis hat dabei keinen Einfluß auf das Stromverhalten, da bei der Hintereinanderschaltung von Wider-ständen der größte Widerstand das Gesamtverhalten maßgeblich beeinflußt.

Da der Stofftransport proportional zum elektrischen Transport verläuft, verringert sich infolge des kleiner werdenden Stroms auch die Abnahme der Leitfähigkeit im Abgeber-kreislauf. Der Versuch wird solange gefahren, bis sich nahezu keine Veränderung der Leitfähigkeit mehr ergibt, d.h. alle Salze aus dem Abgeberkreislauf in den Aufnehmerkreislauf transportiert wurden. Der Sprung im Leitfähigkeitsverlauf im Aufnehmerkreis ergibt sich aus den Temperaturschwankungen bei der Versuchsunterbrechung über Nacht.

Die Veränderungen der Salzkonzentrationen in beiden Kreisläufen für alle Versuche sind in Tabelle 4 angegeben. Bis auf die Entsalzung von A7 konnten alle angesetzten Lösungen hervorragend entsalzt werden. Die letztendlich erreichbare kleinste Konzen-tration ist somit nur noch einige Frage der Zeit, d.h. wie lange der Strom fließen soll.

**Tabelle 4**

| Alkohol | Konzentrat | | | Abgeber | | |
|---|---|---|---|---|---|---|
| | | Anfang | Ende | | Anfang | Ende |
| | Salz | % | % | Salz | % | % |
| A1 | S1 | 1,00 | 10,17 | S1 | 12,0 | 0,0100 |
| A2 | S2 | 0,98 | 19,09 | S2 | 20,0 | 0,9200 |
| A3 | S3 | 1,50 | 4,57 | S3 | 5,0 | 0,0010 |
| A8 | S1 | 1,00 | 9,9 | S1 | 12,0 | 0,1270 |
| A5 | S4 | 0,68 | 13,3 | S4 | 14,0 | 0,0650 |
| A4 | S5 | 2,00 | 6,82 | S5 | 5,0 | 0,0720 |
| A6 | S4 | 0,68 | 8,57 | S4 | 14,0 | 0,0206 |
| A7 | S3 | 1,50 | 4,25 | S3 | 16,5 | 15,2300 |
| A9 | S1 | 1,00 | 5,59 | S1 | 5,0 | 0,0380 |

Anfangs- und Endkonzentrationen im Salzabgeber- sowie Salzaufnehmerkreislauf

Die Selektivität des Salztransportes wird durch die Stromausbeute η beschrieben, d.h. die tatsächlich transportierte Stoffmenge an Salz ($N_{gemessen}$ in Mol) bezogen auf die durch den elektrischen Ladungstransport (elektrischer Strom) maximale mögliche Menge ($N_{theoretisch}$):

$$\eta = N_{gemessen}/N_{theoretisch}$$

Aus den gemessenen Konzentrationen und der Vorlagenmenge und der geflossenen Ladung (Integral aus Strom • Zeit) kann durch genaue Bilanzierung die Stromausbeute berechnet werden. Sie liegt mit Ausnahme des schon erwähnten A7 bei Werten zwischen 0,8 und nahezu 1,0. Bei den Entsalzungen von A3 und A5 wurden nur Werte von ca. 0,6 erreicht.

| Verwendete Chemikalien | | | |
|---|---|---|---|
| Ethandiol (Ethylenglykol) | techn. Ware | 99,9% | Hüls |
| 1,4 Butandiol | techn. Ware | | Hüls |
| Diethylenglykol | | > 99,9% | Riedel-de Haen |
| PEO (MW ca. 1500 g/Mol) | rein | | Fluka |
| Neopentylglykol | rein | > 98,0% | Fluka |
| Glycerin | p.a. | 99,5% | wasserfrei |
| Pentaerythritol | rein | > 97,0% | Fluka |
| 1,3-Dihydroxybenzol (Resorcinol) | rein | > 98,0% | Fluka |
| Polyvinylalkohol | | | Wacker Polyviol G04/140 |
| NaCl | p.a. | | Merck |
| Kaliumacetat | p.a. | | Fluka |
| $Na_2SO_4$ | techn. | > 99,0% | Riedel-de Haen |
| Natriummethylenhexansäure | rein | | Fluka |
| Natriumformiat | p.a. | | Fluka |

Fluka Chemie AG, Industriestr. 25, CH-9471 Buchs
Riedel-de Haën Laborchemikalien GmbH & Co. KG, D-30918 Seelze
Merck KGaA, Frankfurter Str. 250, D-64293 Darmstadt
Wacker-Chemie GmbH, Hanns-Seidel-Platz 4, D-81737 München

**Patentansprüche**

**1.** Verfahren zur Abtrennung mehrfunktioneller Alkohole von wasserlöslichen Salzen aus wäßrigen Systemen, dadurch gekennzeichnet, daß als Trennverfahren ein Elektrodialyseverfahren verwendet wird, mit der Maßgabe, daß die mehrfunktionellen Alkohole nicht Trimethylolpropan umfassen.

**2.** Verfahren nach Anspruch 1, dadurch gekennzeichnet, daß die mehrfunktionellen Alkohole aus Diolen, oligomeren oder polymeren Etherdiolen, Triolen und höherfunktionellen Alkoholen, und polymeren mehrfunktionellen Alkoholen ausgewählt sind.

**3.** Verfahren nach Anspruch 2, dadurch gekennzeichnet, daß die Diole durch die folgende allgemeine Formel dargestellt sind:

$$HO\text{-}R_1\text{-}OH$$

worin $R_1$ eine lineare aliphatische Einheit, eine lineare ungesättigte aliphatische Einheit, eine verzweigte aliphatische Einheit, eine verzweigte ungesättigte aliphatische Einheit, eine cyclische oder alicyclische aliphatische Einheit, eine cyclische oder alicyclische ungesättigte aliphatische Einheit, eine aromatische Einheit, eine heterocyclische aromatische Einheit oder eine Kombination dieser Einheiten bedeutet.

**4.** Verfahren nach Anspruch 2, dadurch gekennzeichnet, daß die Diole Monoglyceride mit der folgenden allgemeinen Formel sind:

$$CH_2OR_r\text{-}CHOR_s\text{-}CH_2OR_t$$

worin zwei der Gruppen $R_r$, $R_s$ und $R_t$ jeweils Wasserstoff bedeuten und die andere Gruppe ist ein gegebenenfalls ungesättigter Monocarbonsäurerest.

**5.** Verfahren nach Anspruch 2,
dadurch gekennzeichnet,
daß die oligomeren oder polymeren Etherdiole durch die folgende allgemeine Formel dargestellt sind:

$$HO\text{-}[(\text{-}CR_2R_3)_n\text{-}O]_m\text{-}H \qquad \text{mit } n \geq 2 \text{ und } m \geq 2$$

worin $R_2$ und $R_3$ jeweils unabhängig voneinander Wasserstoff oder eine aliphatische Gruppe bedeuten.

**6.** Verfahren nach Anspruch 2,
dadurch gekennzeichnet,
daß die Triole und höherfunktionellen Alkohole durch die folgende allgemeine Formel dargestellt sind:

$$R_4(OH)_x \qquad \text{mit } x \geq 3$$

worin $R_4$ eine lineare aliphatische Einheit, eine lineare ungesättigte aliphatische Einheit, eine verzweigte aliphatische Einheit, eine verzweigte ungesattigte aliphatische Einheit, eine cyclische oder alicyclische aliphatische Einheit, eine cyclische oder alicyclische ungesättigte aliphatische Einheit, eine aromatische Einheit, eine heterocyclische aromatische Einheit oder eine Kombination dieser Einheiten bedeutet.

**7.** Verfahren nach Anspruch 2,
dadurch gekennzeichnet,
daß die polymeren mehrfunktionellen Alkohole aus Polyvinylalkohol, Polysacchariden und Polyetherpolyolen der folgenden allgemeinen Formel ausgewählt sind:

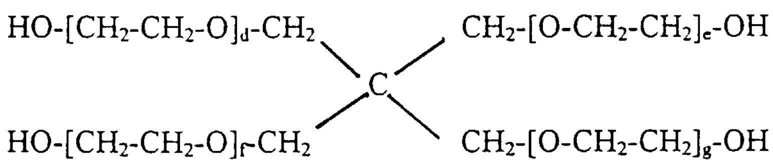

worin d, e, f und g unabhängig voneinander eine ganze Zahl ≥ 1 bedeuten.

**8.** Verfahren nach mindestens einem der Ansprüche 1 bis 7,
dadurch gekennzeichnet,
daß die in dem wäßrigen System enthaltenen Salze und mehrfunktionellen Alkohole in einer Menge bis zu ihrer jeweiligen Sättigungskonzentration vorliegen.

**9.** Verfahren nach mindestens einem der Ansprüche 1 bis 8,
dadurch gekennzeichnet,
daß das Elektrodialyseverfahren bei einer Temperatur im Bereich von 10 bis 50 °C durchgeführt wird.

**10.** Verfahren nach mindestens einem der Ansprüche 1 bis 9,
dadurch gekennzeichnet,
daß das Elektrodialyseverfahren bei einem pH-Wert im Bereich von 4 bis 10 durchgeführt wird.

**11.** Verfahren nach Anspruch 10,
dadurch gekennzeichnet,
daß das Elektrodialyseverfahren bei einem pH-Wert von etwa 7 durchgeführt wird.

**12.** Verfahren nach mindestens einem der Ansprüche 1 bis 11,
dadurch gekennzeichnet,
daß das Elektrodialyseverfahren bei einer Stromdichte im Bereich von 50 bis 750 $A/m^2$ durchgeführt wird.

**13.** Verfahren nach Anspruch 12,
dadurch gekennzeichnet,
daß das Elektrodialyseverfahren bei einer Stromdichte im Bereich von 150 bis 250 $A/m^2$ durchgeführt wird.

**14.** Verfahren nach mindestens einem der Ansprüche 1 bis 13,
dadurch gekennzeichnet,
daß die für die Trennung mittels Elektrodialyse eingesetzten wäßrigen Lösungen zuvor einer Filtration unterworfen wurden.

Figur 1

## Elektrodialysemodul, schematisch

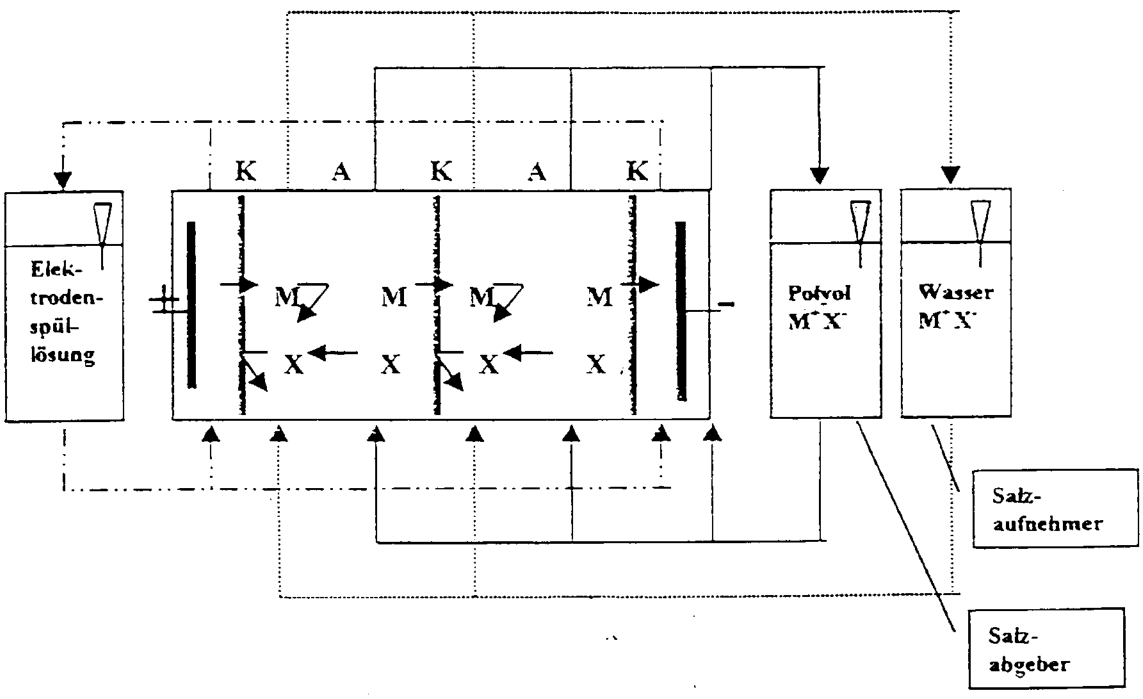

## Figuren 2 und 3

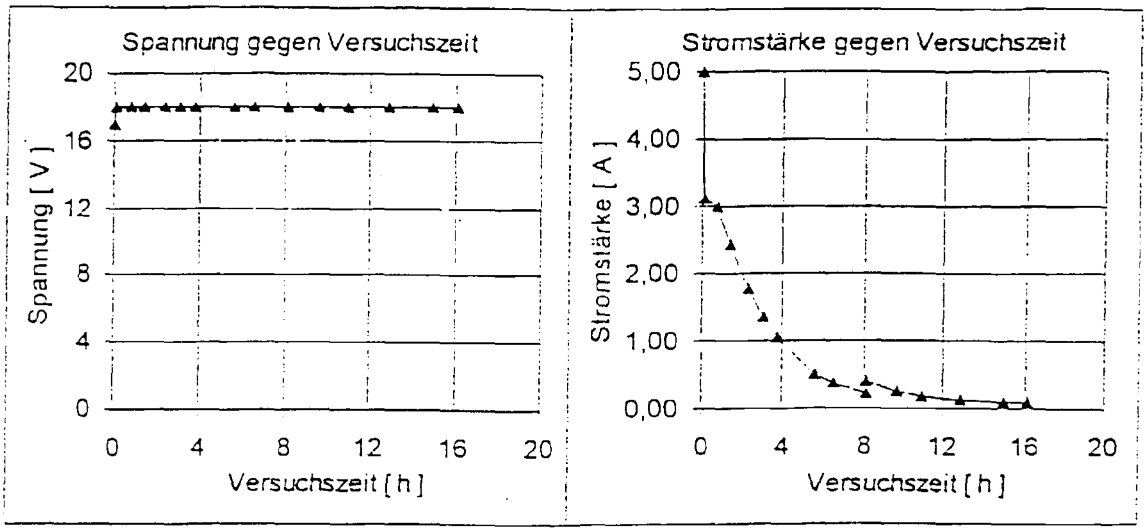


Strom/Spannungsverlauf bei der Entsalzung von A8

## Figuren 4 und 5

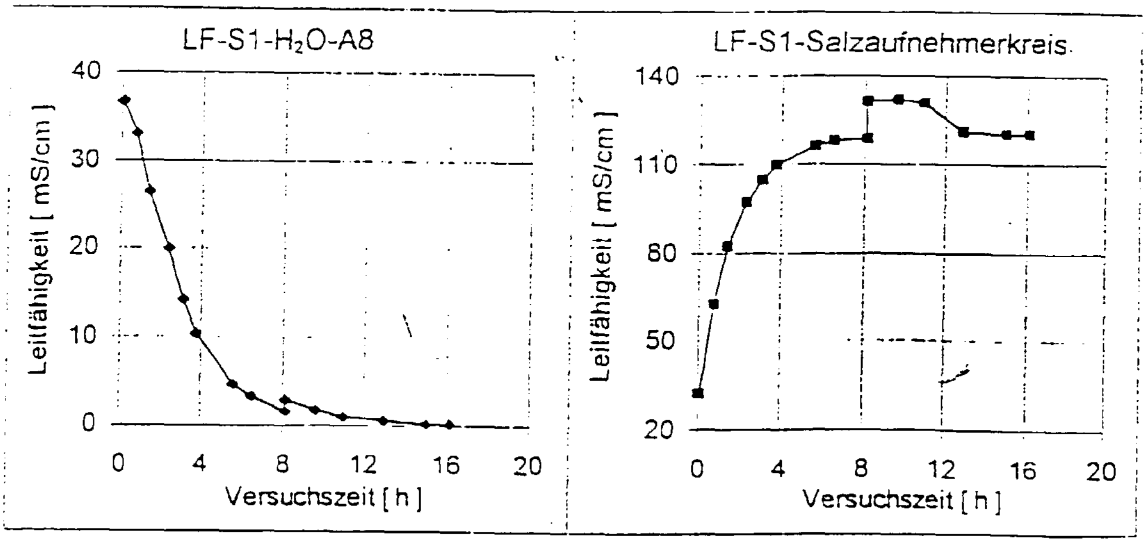


Leitfähigkeitsänderung bei der Entsalzung von A8 im Salzabgeber- (links) und Salzaufnehmerkreislauf (rechts)